# EUROPEAN PATENT APPLICATION

(11) **EP 2 554 207 A1**
(43) Date of publication of application: **06.02.2013**
(21) Application number: 11762627.5
(22) Date of filing: 22.03.2011
(51) Int. Cl.: A61M 5/32, A61M 5/28

(54) **PREFILLED SYRINGE**

(30) Priority: 31.03.2010 JP 2010082583
(71) Applicant: Terumo Kabushiki Kaisha, Tokyo 151-0072 (JP)
(72) Inventor: OKAYAMA ,Tomoki, Nakakoma-gun Yamanashi 409-3853 (JP); OGAWA, Junichi, Nakakoma-gun Yamanashi 409-3853 (JP); OTSU, Shinnosuke, Nakakoma-gun Yamanashi 409-3853 (JP)
(74) Representative: TBK
(86) International application number: PCT/JP2011/056754
(87) International publication number: WO 2011/122395

(57) **Abstract**

A prefilled syringe (1) is provided with an outer tube (2), a medicinal liquid container (14) which can move along the center axis of the outer tube (2), a pressing section (4) which operates and moves the medicinal liquid container (14), a double-ended needle (10), a needle support section (8) which supports the double-ended needle (10), and a seal member (9) which seals between the outer tube (2) and the medicinal liquid container (14). A sealing body (6) which seals the inside of the medicinal liquid container (14) and can slide within the container (14) is built therein. When the pressing section (4) is pressed toward the distal end, after the other end of the double-ended needle (10) is allowed to communicate with the inside of the medicinal liquid container (14), the needle support section (8) makes contact with the sealing body (6) to slide the sealing body (6) toward the side of the proximal end of the medicinal liquid container (14) and is fitted in the medicinal liquid container (14). As a result, the needle support section (8) can move within the outer tube (2). The configuration enables a used puncture needle (10) to be pulled into and contained within the outer tube (2).

## Description

### TECHNICAL FIELD

The present invention relates to a prefilled syringe wherein a medicinal liquid is preliminarily stored in a syringe.

### BACKGROUND ART

In recent years, prefilled syringes having a syringe preliminarily filled with a medicinal liquid have come to be widely used. In some of the generally commercialized prefilled syringes, a needle is preliminarily mounted to a syringe main body (Patent Document 1). In a prefilled syringe in which a needle is not preliminarily mounted, the syringe is provided at its distal end portion with a taper to which a needle hub fitted with a needle is to be mounted. In this case, the needle hub is mounted to the taper at the time of using the prefilled syringe, and, after injection, the syringe main body and the needle hub can be separated from each other and discarded.

However, at the time of separating the needle from the syringe main body after use, a hand may touch the needle or the needle may pierce the hand. Thus, there is a risk of erroneous stinging and infection due to erroneous stinging. Accordingly, there is a need for an improvement in safety of the needle after injection.

### Prior Art Document

### [Patent Documents]

Patent Document 1: Japanese Patent Laid-open No. 2001-187138

### DISCLOSURE OF INVENTION

### Problem to be Solved by the Invention

In view of the foregoing, it is an object of the present invention to provide a prefilled syringe wherein a double-ended needle is preliminarily mounted and a medicinal liquid container is filled with a medicinal liquid, characterized in that the needle after use can be dealt with simply and safely.

### Means for Solving Problem

In order to solve the above problem and attain the above object, according to the present invention, there is provided a prefilled syringe which includes an outer tube, a medicinal liquid container, a pressing section, a double-ended needle, a needle support section, and a seal member.

The outer tube has a distal end and a proximal end.

The medicinal liquid container is a container having an outside diameter smaller than the inside diameter of the outer tube. The medicinal liquid container has a distal opening at the distal end thereof, and the distal opening is sealed with a sealing body, whereby a medicinal liquid is sealed in the inside of the container. In addition, the medicinal liquid container can be moved in the axial direction within the outer tube.

The pressing section is to operate and move the medicinal liquid container.

The double-ended needle is provided at one end thereof with a needlepoint capable of puncturing a living body, and is provided with an end portion capable of puncturing the sealing body.

The needle support section is to support the double-ended needle, is provided in the inside of the outer tube, and can be fitted in the inside of the medicinal liquid container.

The mount section is provided to cover the proximal end face of the outer tube. The double-ended needle is passed through the mount section, and the needle support section is mounted on the side, facing the inside of the outer tube, of the mount section.

The seal member seals between the outer tube and the medicinal liquid container.

In the prefilled syringe according to the present invention, in an initial state, that is, before the prefilled syringe is used, the medicinal liquid container is held in such a position that the other end of the double-ended needle does not puncture the sealing body.

Besides, in a used state, the pressing section is pressed, whereby the medicinal liquid container is moved toward the side of the distal end of the outer tube to cause the other end of the double-ended needle to puncture the sealing body, to slide the sealing body toward the side of the proximal end of the medicinal liquid container, and to fit the needle support section into the inside of the medicinal liquid container.

Further, in a needle accommodation state, the pressing section is pulled, whereby the medicinal liquid container and the needle support section in mutually fitted state are moved together toward the side of the proximal end of the outer tube, and the needlepoint formed at the one end of the double-ended needle is accommodated into the inside of the outer tube.

### Effect of the Invention

According to the present invention, the needle support section is fitted in the medicinal liquid container and is movable within the outer tube. Therefore, by movement of the needle support section toward the side of the proximal end of the outer tube, the double-ended needle can be drawn into the outer tube. As a result, the double-ended needle after use can be accommodated into the space inside the outer tube, simply and safely. Consequently, safety in handling of the prefilled syringe can be secured, and erroneous stinging and infection can be prevented from occurring.

### BRIEF DESCRIPTION OF DRAWINGS

FIG. 1 is a schematic sectional configuration view of a prefilled syringe according to a first embodiment of the present invention, showing a state wherein a protective member has been detached.
FIG. 2 is a schematic sectional configuration view showing a state wherein the protective member is mounted to the prefilled syringe shown in FIG. 1, and showing the same in an unused state.
FIG. 3 shows a schematic sectional configuration view of the prefilled syringe according to the first embodiment of the present invention in use.
FIG. 4 is a schematic sectional configuration view of the prefilled syringe according to the first embodiment of the present invention in use.
FIG. 5 is a schematic sectional configuration view of the prefilled syringe according to the first embodiment of the present invention in use.
FIGS. 6A and 6B are a schematic sectional configuration view of a prefilled syringe according to a second embodiment of the present invention in an unused state, and a plan configuration view of a major part of the same.
FIG. 7 is an exploded perspective view of a major part of the prefilled syringe according to the second embodiment.
FIGS. 8A and 8B are a schematic sectional configuration view of the prefilled syringe according to the second embodiment of the present invention in use, and a plan view of a major part of the same.
FIGS. 9A and 9B are a schematic sectional configuration view of the prefilled syringe according to the second embodiment of the present invention in use, and a plan view of the major part of the same.
FIGS. 10A and 10B are a schematic sectional configuration view of the prefilled syringe according to the second embodiment of the present invention in use, and a plan view of the major part of the same.
FIG. 11 is a schematic sectional configuration view of the prefilled syringe according to the second embodiment of the present invention in use.
FIG. 12 is a schematic sectional configuration view of a prefilled syringe according to a third embodiment of the present invention in an unused state.
FIG. 13 is a schematic sectional configuration view of the prefilled syringe according to the third embodiment of the present invention in use.
FIG. 14 is a schematic sectional configuration view of the prefilled syringe according to the third embodiment of the present invention in use.

### MODE FOR CARRYING OUT THE INVENTION

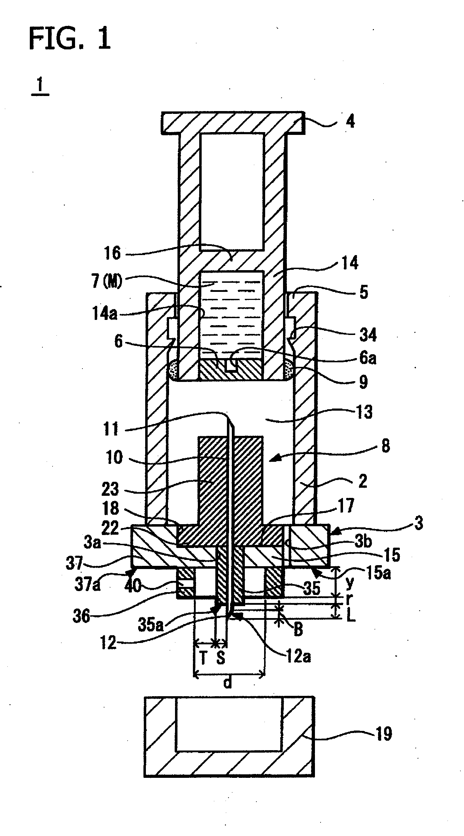

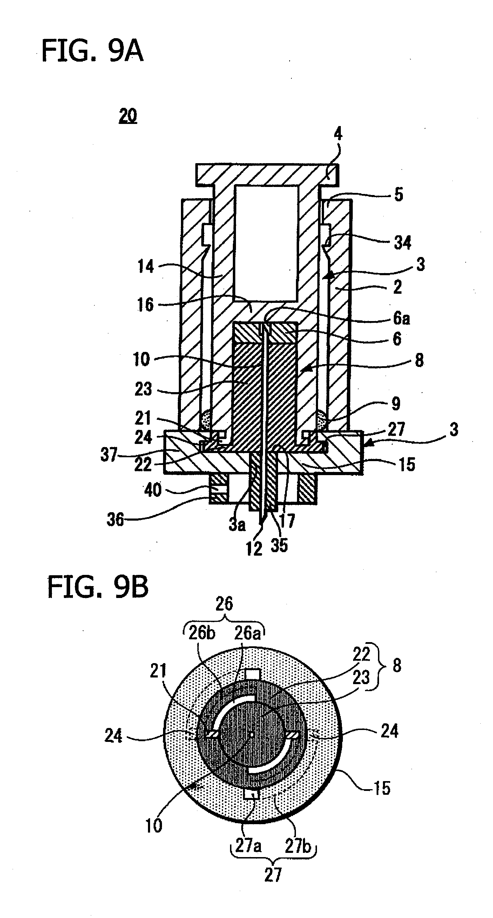

Hereunder, examples of the prefilled syringe according to embodiments of the present invention will be described below, referring to FIGS. 1 to 14. Description of the embodiments of the present invention will be made in the following order. Incidentally, the present invention is not to be restricted to the following examples.
1. First Embodiment: Prefilled Syringe
   1-1 Configuration
   1-2 Using Method
2. Second Embodiment: Prefilled Syringe
   2-1 Configuration
   2-2 Using Method
3. Third Embodiment: Prefilled Syringe
   3-1 Configuration
   3-2 Using Method

### <1. First Embodiment: Prefilled Syringe>

In the first place, a prefilled syringe according to the first embodiment will be described.

### [1-1 Configuration]

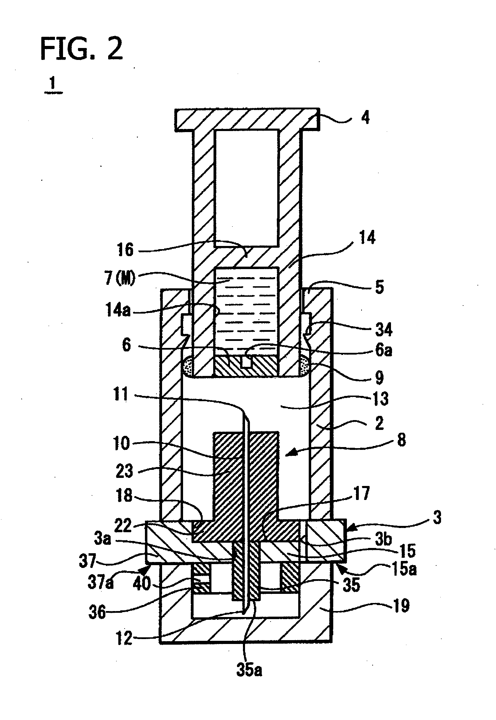

FIG. 1 is a schematic sectional configuration view of a prefilled syringe according to the first embodiment of the present invention, showing a state wherein a protective member has been detached. FIG. 2 is a schematic sectional configuration view showing a state in which the protective member is mounted to the prefilled syringe shown in FIG. 1, and showing the same in an unused state.

As shown in FIG. 1, the prefilled syringe 1 in this embodiment includes an outer tube 2, a medicinal liquid container 14 movable along a center axis of the outer tube 2, a pressing section 4 by which to operate and move the medicinal liquid container 14, a double-ended needle (puncturing section) 10, and a needle support section 8 which supports the double-ended needle 10. In addition, the prefilled syringe 1 includes a mount section 3 on which to mount the needle support section 8, a seal member 9 which seals between the outer tube 2 and the medicinal liquid container 14, a locking claw 34 for preventing the medicinal liquid container 14 from returning, and a stopper section 5 for preventing the medicinal liquid container 14 from slipping off from the outer tube 2. Further, the prefilled syringe 1 is provided with a protective member (cap) 19 for maintaining a sterile state in an unused state of the prefilled syringe 1.

The outer tube 2 is in a tubular shape having a proximal end and a distal end; in this embodiment, the outer tube 2 is composed of a hollow cylindrical member. Incidentally, in the following description, one end side of the outer tube 2 will be referred to as "proximal end," and the other end side as "distal end," and the same applies also in other configurations.

As the material constituting the outer tube 2, for example, various resins may be used. Specific examples of the resins include polyvinyl chloride, polyethylene, polypropylene, cyclic polyolefins, polystyrene, poly(4-methylpentene-1), polycarbonates, acrylic resins, acrylonitrile-butadiene-styrene copolymer, polyesters such as polyethylene terephthalate, etc., butadiene-styrene copolymer, and polyamides (e.g., nylon 6, nylon 6.6, nylon 6.10, nylon 12). Among these resins, preferred are such resins as polypropylene, cyclic polyolefins, polyesters, and poly(4-methylpentene-1), because of their easy moldability. Incidentally, the material forming the outer tube 2 is preferably substantially transparent, in order to secure inside visibility.

The medicinal liquid container 14 is composed of a bottomed tubular member which is provided therein with a bottom portion 16, and is provided on the distal end thereof with a distal opening 14a. The medicinal liquid container 14 is maintained on the proximal end of the outer tube 2, in an unused state. In addition, the outside diameter of the medicinal liquid container 14 is set to be smaller than the inside diameter of the outer tube 2 so that when the medicinal liquid container 14 is moved within the outer tube 2 at the time of use, the inner circumferential surface of the outer tube 2 and the outer circumferential surface of the medicinal liquid container 14 are kept in out-of-contact state.

The material forming the medicinal liquid container 14 is not particularly restricted; for example, the same materials as those for the outer tube 2 mentioned above can be used. Incidentally, the material constituting the medicinal liquid container 14 is preferably substantially transparent, in order to secure inside visibility. In addition, the outer circumferential surface of the medicinal liquid container 14 is formed with graduations (not shown). This enables the user to grasp the amount of a medicinal liquid M stored inside the medicinal liquid container 14.

Besides, a sealing body 6 for sealing the distal opening 14a is held on the distal end in the inside of the medicinal liquid container 14, in an airtight manner. The sealing body 6 is provided to be movable along the inner circumferential surface of the medicinal liquid container 14. The sealing body 6 is so configured that a second needlepoint 11 on the proximal end of the double-ended needle 10 which will be described later can pierce through the sealing body 6.

In addition, the sealing body 6 is formed, in a central area of its surface on the side facing the bottom portion 16 of the medicinal liquid container 14, with a needle accommodating groove 6a continuous with a hole pierced by the second needlepoint 11. The needle accommodating groove 6a is composed of a recessed groove formed to have a predetermined depth from the surface of the sealing body 6. The needle accommodating groove 6a ensures that when the double-ended needle 10 pierces through the sealing body 6, the second needlepoint 11 is accommodated in the needle accommodating groove 6a, without protruding from the surface of the sealing body 6.

The material constituting the sealing body 6 provided in the distal opening 14a of the medicinal liquid container 14 is not specifically restricted. However, it is preferable for the sealing body 6 to be formed from an elastic material, for ensuring good liquid-tightness between the sealing body 6 and the medicinal liquid container 14. Examples of the elastic materials include such elastic materials as various rubber materials such as natural rubber, butyl rubber, isoprene rubber, isobutylene rubber, butadiene rubber, styrene-butadiene rubber, silicone rubbers, etc., various thermoplastic elastomers based on polyurethane, polyester, polyamide, olefin, or styrene, and mixtures thereof.

Incidentally, it suffices that at least an outer circumferential portion of the sealing body 6 is formed from the elastic material as above-mentioned. For example, a configuration may be adopted wherein the sealing body 6 has a core portion (not shown) formed of a resin material, and the above-mentioned elastic material is so disposed as to cover the outer circumference of the core section.

Besides, in a space 7 inside the medicinal liquid container 14 that is surrounded by the bottom portion 16 of the medicinal liquid container 14 and the sealing body 6, the medicinal liquid M is preliminarily stored in a liquid-tight (airtight) manner. This space is a hermetically sealed space which is sealed by the sealing body 6 in an airtight manner, and a sterile state is maintained therein.

Examples of the medicinal liquid M preliminarily stored in the space 7 in the medicinal liquid container 14, in this embodiment, include various vaccines for prevention of various infectious diseases such as influenza, but are not limited to the vaccines. Other examples of the medicinal liquid M than vaccines include carbohydrate injections such as glucose, etc., electrolyte correction injections including electrolyte such as sodium chloride, potassium lactate, etc., vitamin preparations, antibiotic injections, contrast agents, steroid preparations, protease inhibitors, fat emulsions, carcinostatic agents, anesthetic agents, stimulants, narcotics, heparin calcium, and antibody drugs.

In addition, the amount of the medicinal liquid M stored in the space 7 (the volume of the space 7) is not particularly limited; for example, the amount is preferably about 0.02 to 2.0 mL, more preferably about 0.05 to 0.8 mL. In other words, the prefilled syringe 1 is particularly suitable for the case of administering such a small amount of a medicinal liquid M.

The seal member 9 is a member which seals between the outer tube 2 and the medicinal liquid container 14 in an unused state, and which holds the medicinal liquid container 14 on the proximal end of the outer tube 2. The seal member 9 is fixed over the whole circumference of an outer circumferential surface on the distal end of the medicinal liquid container 14. The material forming the seal member 9 is not particularly limited, and the same materials as those for forming the sealing body 6 can be used. In this embodiment, the seal member 9 is composed of an O-ring shaped elastic member. In addition, the seal member 9 may be formed by two-color molding.

An end portion on the outer tube 2 side of the seal member 9 is put in close contact with the inner circumferential surface of the outer tube 2. This ensures that the gap between the inner circumferential surface of the outer tube 2 and the outer circumferential surface of the medicinal liquid container 14 is perfectly sealed, and the medicinal liquid container 14 is held on the proximal end of the outer tube 2 by the seal member 9. With the seal member 9 thus provided between the outer tube 2 and the medicinal liquid container 14, the distal end of the medicinal liquid container 14 is stably held on the proximal end of the outer tube 2 in an unused state. Furthermore, a space 13 inside the outer tube that is surrounded by the outer tube 2, the seal member 9 and the mount section 3 which is described later is sealed in an airtight manner.

The pressing section 4 is a section by which to operate and move the medicinal liquid container 14 along the axial direction (longitudinal direction) of the outer tube 2. The pressing section 4 is composed of a circular disk-like member provided at a proximal portion of the medicinal liquid container 14. The diameter of the pressing section 4 is set to be greater than the diameter of the outer circumference of the outer tube. In this embodiment, the pressing section 4 is formed integrally with the medicinal liquid container 14. While an example wherein the pressing section 4 is formed integrally with the medicinal liquid container 14 is thus shown in this embodiment, this is not restrictive. For example, a configuration may be adopted wherein the medicinal liquid container 14 and the pressing section 4 are composed of separate members, which are joined to each other by adhesion with an adhesive or by fusing or the like.

The double-ended needle 10 is provided on the distal end thereof with a sharp first needlepoint 12 capable of puncturing a living body, and is provided on the proximal end thereof with a sharp second needlepoint 11 capable of piercing through the sealing body 6 of the medicinal liquid container 14. A configuration is secured wherein when the second needlepoint 11 on the proximal end of the double-ended needle 10 pierces through the sealing body 6, the proximal end of the double-ended needle 10 communicates with the inside of the medicinal liquid container 14.

As the double-ended needle 10, those of a size of 22 to 33 gauge (outside diameter: 0.2 to 0.7 mm) according to ISO medical needle pipe standard (ISO9626: 1991/Amd. 1:2001 (E)) can be used. Incidentally, in the case of use in administering into an upper layer part of skin, there can be used needle pipes of a size of 26 to 33 gauge, preferably 30 to 33 gauge.

On the distal end of the first needlepoint 12 of the double-ended needle 10, a cutting edge having a cutting edge surface 12a is formed. The length (hereafter referred to as "bevel length") B of the cutting edge surface 12a on the distal end measured along the axial direction of the double-ended needle 10 is preferably not more than the smallest thickness of the upper layer part of skin (which will be described later) of 1.4 mm (adult). In addition, the bevel length B is preferably not less than about 0.5 mm, which is the bevel length in the case where a 33 gauge double-ended needle is formed with a short bevel. Thus, the bevel length B is preferably set in the range of 0.5 to 1.4 mm. Here, the upper layer part of skin refers to the epidermis and the dermis.

Furthermore, it is more preferable that the bevel length B is not more than the smallest thickenss of the upper layer part of skin of 0.9 mm (child). Thus, the bevel length B is more preferably set in the range of 0.5 to 0.9 mm.

The material forming the double-ended needle 10 is not specifically restricted. Examples of the material include various metallic materials such as stainless steel, aluminum, aluminum alloys, titanium, titanium alloys, Ni-Ti alloys and the like superelastic alloys, as well as various hard resin materials such as polyphenylene sulfide.

The needle support section 8 includes a base section 22, a fixation section 23 fixed to a central portion of a proximal end face of the base section 22, and an adjustment section 35 fixed to a central portion of a distal end face of the base section 22. The needle support section 8 is so mounted that a bottom surface (distal end face) of the base section 22 is in contact with a mount surface 17 of the mount section 3 to be described later and that the adjustment section 35 is passed through a passage hole 3a formed in the mount surface 17. Thus, the mount surface 17 of the mount section 3 is to mount the needle support section 8 thereon by receiving the proximal end face of the base section 22. In the configuration in this embodiment, the base section 22 is formed in a circular disk-like shape, the fixation section 23 is formed in a cylindrical shape, and the base section 22 and the fixation section 23 are formed integrally.

The diameter of the outer circumference of the base section 22 is set to be smaller than the diameter of the inner circumference of the outer tube 2, and, in this embodiment, it is set to be smaller than the diameter of the outer circumference of the medicinal liquid container 14.

The diameter of the outer circumference of the fixation section 23 is set to be equal to or smaller than the inside diameter of the medicinal liquid container 14, and is roughly so sized that the fixation section 23 is held in a close-contact state in the medicinal liquid container 14 at the time of use. Thus, it suffices that the diameter of the outer circumference of the fixation section 23 is so set that the fixation section 23 can be fitted in close contact in the medicinal liquid container 14.

At the time of administering the medicinal liquid M, the proximal end face of the fixation section 23 makes contact with the distal end face of the sealing body 6, and slides the sealing body 6 toward the side of the proximal end of the medicinal liquid container 14, against a distal sliding motion of the medicinal liquid container 14 relative to the outer tube 2 that is effected by pressing the pressing section 4. In other words, the fixation section 23 makes contact with the sealing body 6, and fixes the position of the sealing body 6 relative to the outer tube 2. In addition, the fixation section 23 slides the sealing body 6 toward the side of the proximal end of the medicinal liquid container 14, and is fitted into the inside of the medicinal liquid container 14. In other words, the sliding operation of the sealing body 6 and the operation through which the fixation section 23 is fitted into the inside of the medicinal liquid container 14 are simultaneously performed, and, at the time when the medicinal liquid administration is finished, the fixation section 23 is fitted in the space 7 in which the medicinal liquid M has been stored.

The material forming the base section 22 and the fixation section 23 which constitute the needle support section 8 is not particularly limited. For example, the same materials as those for forming the outer tube 2 mentioned above can be used. In addition, while an example wherein the base section 22 and the fixation section 23 are integrally formed has been shown in this embodiment, a configuration may be adopted in which these sections are formed as separate members and are adhered or fused to each other.

The adjustment section 35 is provided on a central portion of the distal end face of the base section 22 of the needle support section 8, and is configured as a projection projecting in the axial direction of the base section 22. The axis of the adjustment section 35 coincides with the axis of the base section 22. The double-ended needle 10 is piercing from the base section 22 and through the center of the adjustment section 35, and an end face of the adjustment section 35 constitutes a needle protrusion surface 35a from which the first needlepoint 12 side of the double-ended needle 10 protrudes.

The needle protrusion surface 35a is formed as a flat surface orthogonal to the axial direction of the double-ended needle 10. When the double-ended needle 10 is allowed to puncture an upper layer part of skin, the needle protrusion surface 35a makes contact with the surface of the skin, to determine the depth of puncture of the double-ended needle 10. In other words, the depth to which the double-ended needle 10 punctures the upper layer part of skin is determined by the length by which the double-ended needle 10 protrudes from the needle protrusion surface 35a (this length will hereafter be referred to as "protrusion length L").

The thickness of the upper layer part of skin corresponds to the depth from the surface of skin to a dermis layer, and is generally in the range of 0.5 to 3.0 mm. Accordingly, the protrusion length L of the double-ended needle 10 can be set in the range of 0.5 to 3.0 mm.

Meanwhile, vaccines are generally administered into a brachial region, and, in the case of administration into an upper layer part of skin, it is considered suitable for the vaccine to be administered into a peripheral region of shoulder where the skin is thick, particularly into a deltoid region. In view of this, 19 children and 31 adults were subjected to measurement of the thickness of a skin upper layer part of the deltoid muscle. The measurement was conducted by sonography-imaging the skin upper layer part where ultrasonic wave reflectance is high, by use of an ultrasonic measuring instrument (NP60R-UBM, a high-resolution echoscope for small animals, produced by Nepa Gene Co., Ltd.). Incidentally, since the measurement values were in log-normal distribution, the range of MEAN±2SD was determined by use of geometric mean.

As a result, the thickness of the skin upper layer part of the deltoid muscle in children was found to be 0.9 to 1.6 mm. Besides, the thickness of the skin upper layer part of the deltoid muscle in adults was found to be 1.4 to 2.6 mm in distal part, 1.4 to 2.5 mm in central part, and 1.5 to 2.5 mm in proximal part. From these results, it was confirmed that the thickness of the skin upper layer part of the deltoid muscle is not less than 0.9 mm in the cases of children, and not less than 1.4 mm in the cases of adults. Accordingly, in injection into a skin upper layer part of the deltoid muscle, the protrusion length L of the double-ended needle 10 is preferably set in the range of 0.9 to 1.4 mm.

With the protrusion length L set in this manner, the cutting edge surface 12a of the first needlepoint 12 can be located assuredly in the upper layer part of skin. As a result, a needle hole (medicinal liquid outlet) opening at the cutting edge surface 12a can be located in the upper layer part of skin, irrespectively of at which position in the cutting edge surface 12a the needle hole is located. Incidentally, even when the medicinal liquid outlet is located in the upper layer part of skin, if the first needlepoint 12 pierces too deeply into the upper layer part of skin, the medicinal liquid M would be seeped under the subcutaneous region through the part between the side surface of an end portion of the first needlepoint 12 and the incised skin. Therefore, it is important for the cutting edge surface 12a to be located assuredly in the upper layer part of skin.

Incidentally, in the case of use for administration into an upper layer part of skin, it is difficult to set the bevel length B to or below 1.0 mm, for double-ended needles of more than 26 gauge in diametral size. Therefore, in order to set the protrusion length L of the first needlepoint 12 of the double-ended needle 10 in the preferable range (0.9 to 1.4 mm), it is preferable to use a double-ended needle smaller than 26 gauge in diametral size.

The needle protrusion surface 35a is formed so that the distance S from the circumferential edge thereof to the circumferential surface of the double-ended needle 10 is not more than 1.4 mm, and preferably in the range of 0.3 to 1.4 mm. The distance S from the circumferential edge of the needle protrusion surface 35a to the circumferential surface of the double-ended needle 10 is set taking into account the fact that a pressure is exerted on a blister formed by administration of the medicinal liquid M into the upper layer part of skin. Specifically, the needle protrusion surface 35a is set to a size which is sufficiently smaller than the blister formed in the upper layer part of skin and which, therefore, does not obstruct the formation of the blister. Consequently, it is possible to prevent a situation in which the needle protrusion surface 35a would press the skin in the surroundings of the double-ended needle 10 to cause leakage of the medicinal liquid administered.

The mount section 3 includes a substantially circular disk-shaped mount section main body 15, a stabilization section 36, and a guide section 37 serving as a pressing yardstick section, and is provided with the passage hole 3a through which the adjustment section 35 can be passed. The mount section main body 15 is formed in a circular disk-like shape sized to cover the distal end face of the outer tube 2, and is provided fixedly on the distal end face of the outer tube 2. The proximal end face of the mount section main body 15 is formed with a circular recess 18, and a bottom surface (mount surface) 17 of the recess 18 is formed as a horizontal surface orthogonal to the center axis of the outer tube 2. The diameter of the recess 18 in the mount section 3 is set to be a diameter as large as the diameter of the base section 22 so that the base section 22 can be fitted in the recess 18 in the mount section 3. In addition, the depth of the recess 18 (namely, the thickness of the mount section main body 15 from its proximal end face to its mount surface) is set to be approximately equal to the thickness of the base section 22 from its distal end face to its proximal end face. The passage hole 3a piercing through the mount section 3 is provided in a central portion of the mount surface 17. In an unused state, the base section 22 constituting the needle support section 8 is provided and held in the recess in the mount section with fitting each other, and the adjustment section 35 is passed through the passage hole 3a.

In addition, the mount section main body 15 is provided with a first vent hole 3b piercing therethrough from the proximal end face to the distal end face thereof. This vent hole 3b establishes communication between the space 13 inside the outer tube and the exterior of the outer tube 2. Furthermore, the first vent hole 3b may be fitted with a filter, for enhancing the maintenance of sterile state in the space 13 inside the outer tube.

The stabilization section 36 is provided on the distal end face 15a of the mount section main body 15. The stabilization section 36 is formed in a tubular shape continuous with a circumferential edge portion of the distal end face 15a. In a tube hole of the stabilization section 36, there are disposed the first needlepoint 12 of the double-ended needle 10 and the adjustment section 35. In other words, the stabilization section 36 is formed in a tubular shape covering the periphery of the adjustment section 35 penetrated by the double-ended needle 10.

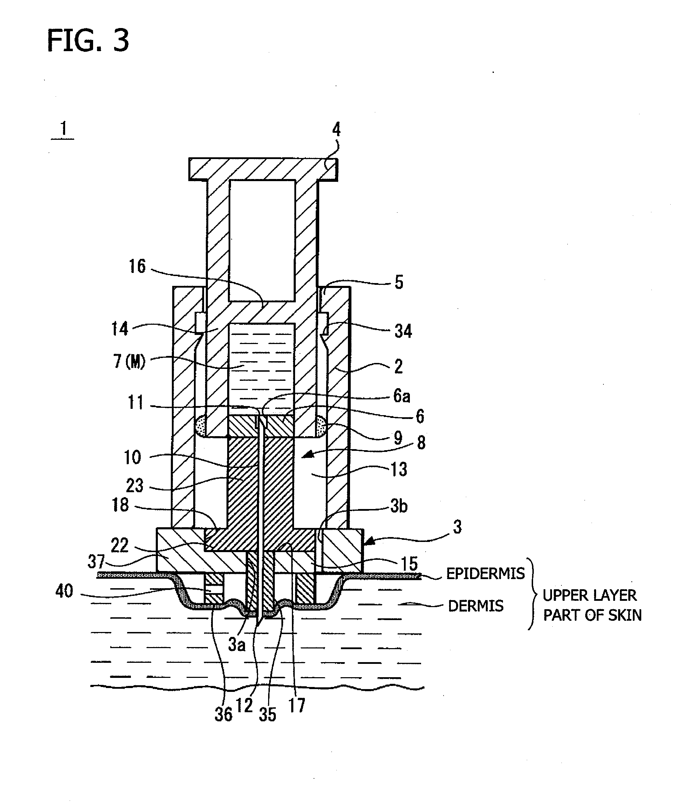

Besides, when the first needlepoint 12 of the double-ended needle 10 is allowed to puncture a living body, as shown in FIG. 3, the needle protrusion surface 35a makes contact with the surface of a skin, and an end face 36a of the stabilization section 36 also makes contact with the surface of the skin. In this instance, the contact of the end face 36a of the stabilization section 36 with the skin stabilizes the prefilled syringe 1, whereby the double-ended needle 10 can be kept in the posture of being substantially perpendicular to the skin.

Incidentally, the end face 36a of the stabilization section 36 may be located on the same plane as the needle protrusion surface 35a, or may be located on the side of the first needlepoint 12 of the double-ended needle 10 relative to the needle protrusion surface 35a. In either case, the double-ended needle 10 can be kept in the posture of being substantially perpendicular to the skin. Incidentally, taking into account the protuberance of the skin upon pressing of the stabilization section 36 against the skin, the axial distance r between the end face 36a of the stabilization section 36 and the needle protrusion surface 35a is preferably set to be not more than 1.3 mm.

In addition, the inside diameter d of the stabilization section 36 is set to be equal to or greater than the diameter of the blister formed in the skin. Specifically, the inside diameter d is so set that the distance T from the inner wall surface of the stabilization section 36 to the circumferential edge of the needle protrusion surface 35a is in the range of 4 to 15 mm. This makes it possible to prevent the formation of the blister from being obstructed by application of a pressure to the blister from the inner wall surface of the stabilization section 36.

The distance T from the inner wall surface of the stabilization section 36 to the circumferential edge of the needle protrusion surface 35a does not have any particular upper limit, insofar as it is not less than 4 mm. If the distance T is enlarged, however, the outside diameter of the stabilization section 36 is enlarged, so that in the case of puncturing a slender arm such as a child's arm with the double-ended needle 10, it is difficult to bring the whole part of the end face 36a of the stabilization section 36 into contact with the skin. Therefore, the distance T is preferably determined to be 15 mm at maximum, taking into account the slenderness of the child's arm.

In addition, where the distance S from the circumferential edge of the needle protrusion surface 35a to the circumferential surface of the double-ended needle 10 is not less than 0.3 mm, the adjustment section 35 would not enter into the skin. Therefore, taking into account the distance T (not less than 4 mm) from the inner wall surface of the stabilization section 36 to the circumferential edge of the needle protrusion surface 35a and the diameter (about 0.3 mm) of the needle protrusion surface 35a, the inside diameter d of the stabilization section 36 can be set to be not less than 9 mm.

Furthermore, the stabilization section 36 is formed with a second vent hole 40 penetrating the stabilization section 36 from its outer circumferential surface to its inner circumferential surface. With the second vent hole 40 thus provided in the stabilization section 36, it is ensured that when the stabilization section 36 is set in contact with a skin, as shown in FIG. 3, the space surrounded by the stabilization section 36 and the skin and the space outside the stabilization section 36 can be allowed to communicate with each other. Besides, the first vent hole 3b and the second vent hole 40 constitute a ventilation means for opening the space 13 inside the outer tube to the exterior.

Incidentally, the shape of the stabilization section 36 is not restricted to the cylindrical shape; for example, the stabilization section 36 may be formed in a polygonal tubular shape, such as a tetragonal prism or hexagonal prism provided with a tube hole in the center thereof.

The guide section 37 is provided at a side surface portion of the mount section main body 15. The guide section 37 is formed as a flange having a ring-like shape projecting outward in the radial direction of the mount section main body 15 from a side surface portion of the mount section main body 15. In addition, the guide section 37 is projecting substantially perpendicularly to the outer circumferential surface of the stabilization section 36.

Furthermore, the guide section 37 has a contact surface 37a coming into contact with a skin. The contact surface 37a is a flat surface which is substantially parallel to the end face 36a of the stabilization section 36. With the stabilization section 36 pressed against a skin until the contact surface 37a of the guide section 37 makes contact with the skin, the force with which the stabilization section 36 and the double-ended needle 10 press the skin can always be secured to be not less than a predetermined value. This ensures that the part (corresponding to the protrusion length L), protruding from the needle protrusion surface 35a, of the double-ended needle 10 is made to puncture the skin assuredly.

In addition, the distance from the contact surface 37a of the guide section 37 to the end face 36a of the stabilization section 36 is set to such a length that the double-ended needle 10 can puncture a skin, with the stabilization section 36 and the double-ended needle 10 pressed against the skin with an appropriate pressing force. Hereafter, this length will be referred to as "guide section height y."

Incidentally, the appropriate pressing force with which the double-ended needle 10 and the stabilization section 36 press the skin is, for example, 3 to 20 N. As a result, the pressing force exerted on the skin by the double-ended needle 10 and the stabilization section 36 can be guided for the user by the guide section 37, and the first needlepoint 12 and the cutting edge surface 12a of the double-ended needle 10 can be assuredly located in the upper layer part of skin, which is effective in making the user feel safe.

Specifically, in the case where the inside diameter d of the stabilization section 36 is in the range of 11 to 14 mm, the guide section height y is appropriately set based on the length x (hereafter referred to as guide section length x) from the projecting end surface of the guide section 37 to the outer circumferential surface of the stabilization section 36. For instance, in the case where the inside diameter d of the stabilization section 36 is 12 mm, the guide section height y is set in the range of 2.3 to 6.6 mm when the guide section length x is 3.0 mm, for example.

Besides, in this embodiment, in the initial state, the base section 22 of the needle support section 8 is fitted and held in the recess 18 in the mount section 3, and the first needlepoint 12 on the distal end of the double-ended needle 10 is passed through the mount section 3. Therefore, the needle support section 8 for supporting the double-ended needle 10 is stably held inside the outer tube 2. In addition, the mount surface 17 of the mount section 3 is a horizontal surface orthogonal to the center axis of the outer tube 2, and the distal end face (bottom surface) of the base section 22 of the needle support section 8 is held in contact with the mount surface 17. Accordingly, the double-ended needle 10 can be prevented from being inclined.

The material forming the mount section main body 15, the adjustment section 35, the stabilization section 36, and the guide section 37 of the mount section 3 is not specifically restricted. For example, the same materials as those for forming the outer tube 2 mentioned above can be used. In addition, the adjustment section 35 and the stabilization section 36 are provided on the side of the distal end of the mount section main body 15. The adjustment section 35 and the stabilization section 36 may be formed integrally with the mount section main body 15, or may be formed separately from the mount section main body 15 and adhered to the mount section main body 15.

The locking claw 34 is projectingly provided at an inner circumferential surface of the outer tube 2. Specifically, the locking claw 34 is formed at the inner circumferential surface of the outer tube 2 on the proximal end relative to a position at which the tip of the seal member 9 makes contact with the inner circumferential surface of the outer tube 2 in an unused state. The locking claw 34 is so formed that, when the medicinal liquid container 14 is pulled toward the proximal end from the position in the unused state, the seal member 9 rides over the locking claw 34. When the medicinal liquid container 14 is operated and moved within the outer tube 2 so that the seal member 9 comes to the proximal end relative to the locking claw 34, the seal member 9 is locked on the proximal end of the locking claw 34. This ensures that, after the seal member 9 is locked by the locking claw 34, the medicinal liquid container 14 is prevented from moving toward the distal end under its own weight.

The stopper section 5 is formed at the proximal end face of the outer tube 2, in such a manner as to reduce the gap between the inner circumferential surface of the outer tube 2 and the outer circumferential surface of the medicinal liquid container 14. The stopper section 5 is provided for preventing the medicinal liquid container 14 from slipping off to the proximal end of the outer tube 2 after the seal member 9 is locked on the locking claw 34. The stopper section 5 is so configured that the seal member 9 cannot pass to the proximal end beyond the stopper section 5.

While an example wherein the locking claw 34 and the stopper section 5 are formed integrally with the outer tube 2 is shown in this embodiment, this is not restrictive. For example, a configuration may be adopted wherein the locking claw 34 and the stopper section 5 are formed as members separate from the outer tube 2 and are joined to the outer tube 2 by adhesion with an adhesive or by fusing or the like.

As shown in FIG. 2, the protective member 19 is a member for sealing a space surrounding the first needlepoint 12, on the side of puncturing a living body, of the double-ended needle 10. In the unused state, the protective member 19 is detachably mounted to the stabilization section of the needle support section 8.

The protective member 19 is formed in a bottomed tubular shape having a bottom section on the distal end; in this embodiment, it is formed in a bottomed cylindrical shape. In the unused state, with a side surface of the protective member 19 mounted onto an outer circumferential portion of the mount section 3, the inside of the protective member 19 is hermetically sealed, whereby the sterile state in the space 13 inside the outer tube and the inside of the protective member 19 is maintained.

At the time of using the prefilled syringe 1, the protective member 19 is detached from the needle support section 8 as shown in FIG. 1, whereby sealing of the first needlepoint 12, on the side of puncturing a living body, of the double-ended needle 10 is released.

The material forming the protective member 19 is not particularly limited. For example, the same materials as those for forming the outer tube 2 or those for forming the sealing body 6 mentioned above can be used.

Incidentally, in this prefilled syringe 1, after the protective member 19 is mounted onto the distal end of the mount section 3 and required parts are sterilized, the sterile state in the space 13 inside the outer tube and the inside of the protective member 19 is maintained as above-mentioned, and the sterile state of the double-ended needle 10 is maintained.

### [1-2 Using Method]

Next, an example of the method of using the prefilled syringe 1 according to this embodiment will be described below. FIGS. 3 to 5 show schematic sectional configuration views of the prefilled syringe 1 in use.

At the time of using the prefilled syringe 1, first, as shown in FIG. 1, the protective member 19 is detached from the distal end of the needle support section 8 of the prefilled syringe 1 prepared. This completes the preparation for administration of a medicinal liquid. Thus, in this prefilled syringe 1, preparation for administration of a medicinal liquid can be conducted simply and speedily. Then, the end face 36a of the stabilization section 36 is allowed to face a skin. This permits the first needlepoint 12 of the double-ended needle 10 to face the skin to be punctured. Next, as shown in FIG. 3, the prefilled syringe 1 is moved substantially perpendicularly to the skin to let the double-ended needle 10 puncture the skin, and the end face 36a of the stabilization section 36 is pressed against the skin.

Here, the needle protrusion surface 35a of the adjustment section 35 and the end face 36a of the stabilization section 36 are located on the same plane. This ensures that the needle protrusion surface 35a of the adjustment section 35 makes contact with the skin, whereby the skin can be deformed to be flat, and the first needlepoint 12 of the double-ended needle 10 can puncture the skin by the protrusion length L.

Subsequently, the stabilization section 36 is pressed against the skin until the contact surface 37a of the guide section 37 makes contact with the skin. Here, the guide section height y is set to such a length that the double-ended needle 10 can puncture the skin, with the double-ended needle 10 and the stabilization section 36 pressed against the skin with an appropriate pressing force. This ensures that the force with which the skin is pressed by the stabilization section 36 is brought to a predetermined value. Therefore, the pressing force of the stabilization section 36 can be guided for the user, and the stabilization section 36 can be pressed against the skin with an appropriate pressing force. Consequently, the first needlepoint 12 and the cutting edge surface 12a of the double-ended needle 10 can be assuredly located in an upper layer part of skin.

With the guide section 37 thus serving as a yardstick for guiding the pressing force of the stabilization section 36, the first needlepoint 12 of the double-ended needle 10 can be assuredly located in the upper layer part of skin. Accordingly, the medicinal liquid can be reliably administered into the upper layer part of skin, and the user's feeling of security can be enhanced.

In addition, the contact of the stabilization section 36 with the skin makes it possible to stabilize the double-ended needle 10, and to let the first needlepoint 12 puncture the skin straight. Consequently, unintentional movement of the double-ended needle 10 can be prevented from occurring, and stable administration of the medicinal liquid can be performed.

Furthermore, in the case of a needle having an extremely small protrusion length of about 0.5 mm, for example, the first needlepoint 12 put into contact with a skin may fail to puncture the skin. However, when the stabilization section 36 is pressed against a skin and the skin is pressed down in the vertical direction, the skin on the inner side of the stabilization section 36 is stretched, resulting in the condition where a tension is applied to the skin. Therefore, it becomes difficult for the skin to escape from the first needlepoint 12 of the double-ended needle 10, and, accordingly, it becomes easier for the first needlepoint 12 to pierce the skin.

Besides, since the protrusion length L is set in the range of 0.5 to 3.0 mm, the first needlepoint 12 and the cutting edge surface 12a of the double-ended needle 10 are assuredly located in the upper layer part of skin. The adjustment section 35 is fixed in close contact with the surroundings of the double-ended needle 10, and it is ensured that no gap is generated between the adjustment section 35 and that portion of the double-ended needle 10 which penetrates the adjustment section 35.

Therefore, when the needle protrusion surface 35a of the adjustment section 35 is put in contact with a skin, the skin in the surroundings of the double-ended needle 10 can be deformed to be flat. As a result, the double-ended needle 10 can be made to puncture the skin by the protrusion length L, and the first needlepoint 12 of the double-ended needle 10 can be assuredly located in the upper layer part of skin. Next, the user presses the pressing section 4, whereby the medicinal liquid container 14 with the medicinal liquid M confined therein is slid along the axial direction of the outer tube 2. Then, the fixation section 23 of the needle support section 8 makes contact with the sealing body 6. In this instance, the second needlepoint 11 of the double-ended needle 10 is allowed to puncture the sealing body 6. As a result, passage of the medicinal liquid M stored in the medicinal liquid container 14 into the double-ended needle 10 is completed.

In this embodiment, since the sealing body 6 is formed with the needle accommodating groove 6a, the second needlepoint 11 is accommodated in the needle accommodating groove 6a.

In addition, the stabilization section 36 is provided with the second vent hole 40. At the time of operating and moving the pressing section 4, the pressure in the space surrounded by the stabilization section 36 and the skin can be prevented from being raised.

Subsequently, the pressing section 4 is pressed toward the needle support section 8 of the outer tube 2, whereon the sealing body 6 is pushed into the medicinal liquid container 14 by the fixation section 23. Then, the sealing body 6 is slid within the medicinal liquid container 14 along the axial direction of the latter, whereby the medicinal liquid M in the medicinal liquid container 14 is discharged through the second needlepoint 11 of the double-ended needle 10 and through the first needlepoint 12 into the living body, to be administered into a target part.

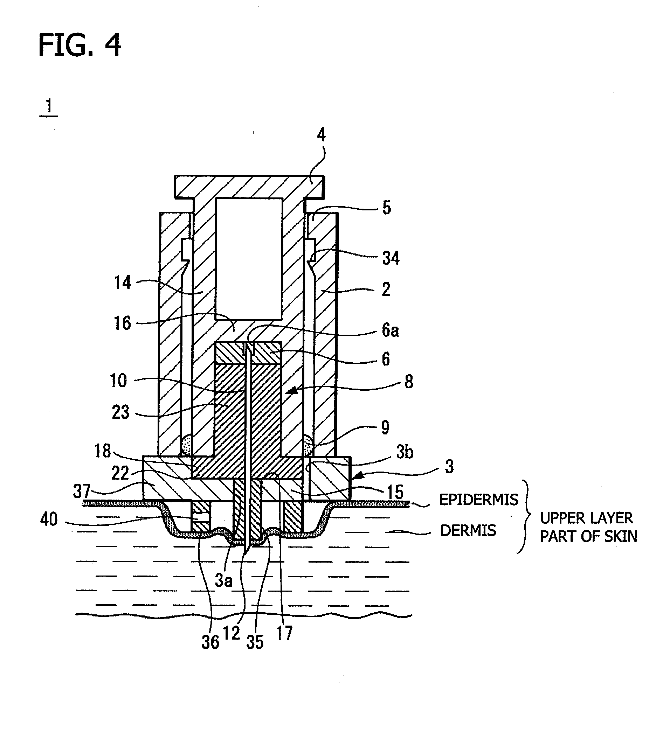

Thus, at the time of medicinal liquid administration (in other words, in a used state), the fixation section 23 slides the sealing body 6 toward the side of the proximal end of the medicinal liquid container 14, so that the fixation section 23 is gradually inserted into the inside of the medicinal liquid container 14. Then, the sealing body 6 comes into contact with the bottom portion 16 of the medicinal liquid container 14, whereby the medicinal liquid administration is completed. In this instance, the second needlepoint 11 is accommodated in the needle accommodating groove 6a in the sealing body 6 and, therefore, does not protrude from the surface of the sealing body 6 to the side of the bottom portion 16 of the medicinal liquid container 14. Therefore, the sealing body 6 makes contact with the bottom portion 16 of the medicinal liquid container 14, without leaving any gap therebetween. When the sealing body 6 comes into contact with the bottom portion 16 of the medicinal liquid container 14 and the medicinal liquid administration is thereby completed, as shown in FIG. 4, the fixation section 23 is in the state of being fitted in that part of the medicinal liquid container 14 in which

the medicinal liquid M has been stored.

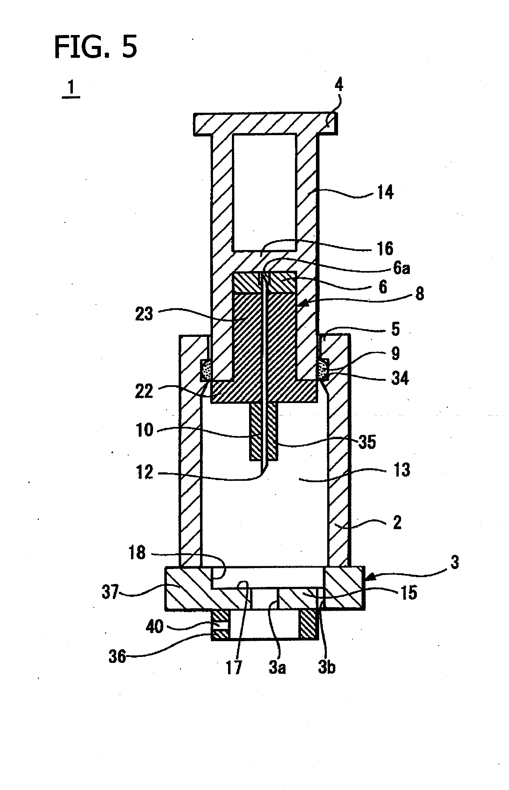

After the medicinal liquid administration is finished, the first needlepoint 12 having been puncturing the living body is drawn out of the living body. Then, the pressing section 4 is pulled toward the proximal end relative to the outer tube 2, whereby the medicinal liquid container 14 is operated and moved toward the side of the proximal end of the outer tube 2. This results in that, since on the distal end of the medicinal liquid container 14 the fixation section 23 having been inserted at the time of medicinal liquid administration is present in the state of being fitted in the inside of the medicinal liquid container 14, as shown in FIG. 5, the medicinal liquid container 14 is moved toward the side of the proximal end of the outer tube 2 and, simultaneously, the needle support section 8 is also moved toward the side of the proximal end of the outer tube 2. As a result, the double-ended needle 10 is drawn out of the mount section 3, and is drawn into the space 13 inside the outer tube. Eventually, the first needlepoint 12 of the double-ended needle 10 that has punctured the living body is accommodated into the space 13 inside the outer tube, whereby the prefilled syringe 1 is put in a needle accommodation state.

Then, the pressing section 4 is pulled toward the proximal end until the seal member 9 is disengaged from the locking claw 34 therethrough. At the time when the seal member 9 has just been disengaged from the locking claw 34, the operation of moving the medicinal liquid container 14 is stopped. After the seal member 9 is disengaged from the locking claw 34, the seal member 9 is locked on the proximal end of the locking claw 34, so that the medicinal liquid container 14 would not return toward the distal end under its own weight. In addition, since the proximal end face of the outer tube 2 is provided with the stopper 5 through which the seal member 9 cannot pass, the medicinal liquid container 14 is also prevented from slipping off to the proximal end of the outer tube 2.

As has been described above, in this embodiment, the double-ended needle 10 drawn out of the living body can be safely accommodated into the space 13 inside the outer tube, by only the operation of pulling the pressing section 4. Therefore, erroneous stinging after use can be prevented from occurring, and, therefore, infection and the like can be prevented from occurring. In addition, there is no need for an operation in which the user's hand must be brought close to the first needlepoint 12 of the double-ended needle 10, such as an operation of putting a protector onto the double-ended needle 10, after use. Accordingly, the user can deal with the prefilled syringe 1 safely.

Besides, in this embodiment, the presence of the locking claw 34 ensures that after the double-ended needle 10 after use is accommodated into the space 13 inside the outer tube, the first needlepoint 12 of the double-ended needle 10 is prevented from being again exposed to the exterior of the outer tube 2. This enables the double-ended needle 10 to be safely dealt with after use.

Meanwhile, in the prefilled syringe 1 in this embodiment, a configuration is adopted wherein the fixation section 23 as a part of the needle support section 8 is fitted in the inside of the medicinal liquid container 14 and the needle support section 8 is pulled up toward the side of the proximal end of the outer tube 2. Therefore, the fitting force between the medicinal liquid container 14 and the fixation section 23 needs be set to be somewhat greater than the fitting force between the mount section 3 and the base section 22. In addition, the fixation section 23 and the medicinal liquid container 14 need be fitted to each other with such a fitting force that, after the fixation section 23 is fitted in the inside of the medicinal liquid container 14, the fixation section 23 would not slip off from the inside of the medicinal liquid container 14 under its own weight.

Accordingly, the fixation section 23 and the medicinal liquid container 14 need be so designed that the fixation section 23 is fitted in a close-contact state in the inside of the medicinal liquid container 14. This is contrasted to the fact that it suffices for the mount section 3 and the base section 22 to be so designed that they are fitted to each other in such a fashion that the base section 22 would not be inclined or tilted down.

In this embodiment, the fitting force between the mount section 3 and the base section 22 can be regulated by the diameter of the recess 18 and the diameter of the outer circumference of the base section 22. Besides, the fitting force between the fixation section 23 and the medicinal liquid container 14 can be regulated by the diameter of the outer circumference of the fixation section 23 and the diameter of the inner circumference of the medicinal liquid container 14. Therefore, by appropriately determining these diameters, the prefilled syringe 1 according to this embodiment can be configured.

In addition, according to the prefilled syringe 1 in this embodiment, the medicinal liquid container 14 with a required amount of a medicinal liquid M stored therein is provided, so that wasting of the medicinal liquid can be obviated, and economy is secured.

Besides, the medicinal liquid container 14 and the double-ended needle 10 are preliminarily disposed, and, in the unused state, the sterile state of the space 13 inside the outer tube in which the double-ended needle 10 is disposed is maintained. Therefore, preparation for administration of the medicinal liquid can be performed simply and speedily, by only an operation of detaching the protective member 19.

While the prefilled syringe 1 to be used for administration of a medicinal liquid into an upper layer part of skin has been shown as an example in this embodiment, the prefilled syringe 1 according to the present invention is applicable to various uses such as hypodermic administration, intramuscular administration and intravascular administration. In that case, it suffices that the protrusion length L of the double-ended needle 10 and the like are set correspondingly to the various uses. In addition, the configurations of the stabilization section 36, the adjustment section 35, and the guide section 37 may be omitted. Besides, while a configuration wherein the first needlepoint 12 of the double-ended needle 10 is completely drawn into the space 13 inside the outer tube has been adopted in this embodiment, a configuration suffices wherein the needle support section 8 is retracted to such an extent that the cutting edge surface of the first needlepoint 12 is not exposed from the puncture surface (for example, the distal end face of the mount section 3).

### <2. Second Embodiment: Prefilled Syringe>

Next, a prefilled syringe according to a second embodiment of the present invention will be described below.

### [2-1 Configuration]

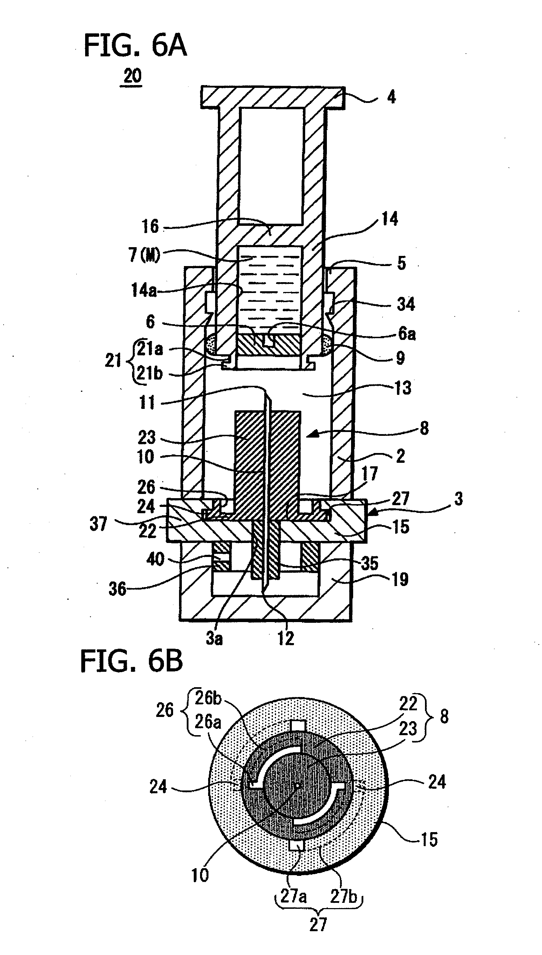

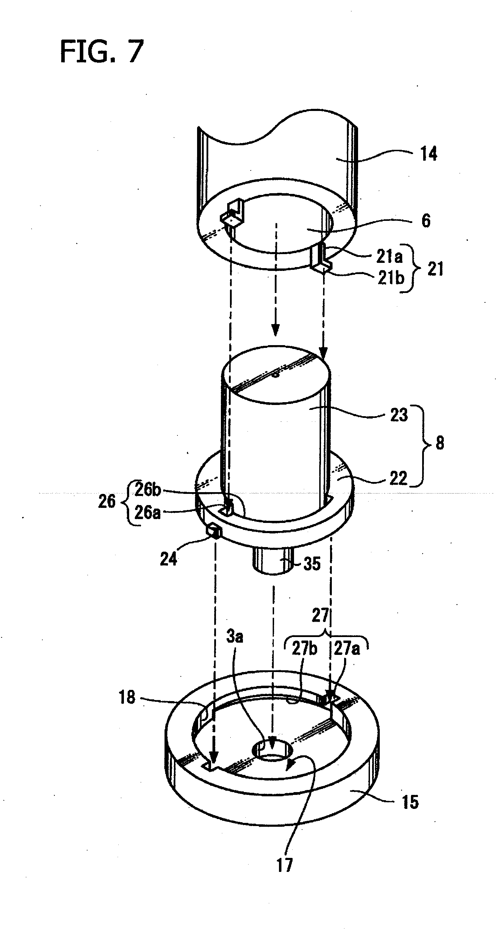

First, the configuration of the prefilled syringe according to this embodiment will be described. FIG. 6A is a schematic sectional configuration view showing a prefilled syringe 20 according to this embodiment, in an unused state, and FIG. 6B is a plan configuration view showing a mount section 3 and a needle support section 8 in an extracting manner. FIG. 7 is an exploded perspective view of the mount section 3, the needle support section 8, and a medicinal liquid container 14 of the prefilled syringe 20 according to this embodiment. In FIGS. 6A, 6B and 7, the parts corresponding to those in FIG. 1 are denoted by the same letters or numerals as used above, and overlapping descriptions of the parts will be omitted. Besides, in FIG. 7, a double-ended needle 10 is omitted from drawing.

The prefilled syringe 20 in this embodiment has a first connection section by which the needle support section 8 and the medicinal liquid container 14 can be connected to and disconnected from each other, and a second connection section by which the needle support section 8 with the medicinal liquid container 14 can be connected to and disconnected from each other.

The first connection section is composed of first fitting claws 21 formed at a distal end face of the medicinal liquid container 14, and first fitting holes 26 formed in a proximal end face of a base section 22 of the needle support section 8.

The second connection section is composed of second fitting claws 24 formed at a side surface of the base section 22 of the needle support section 8, and second fitting holes 27 formed in a side surface of a recess 18 in the mount section 3.

As shown in FIG. 7, the first fitting claws 21 are provided in opposed two locations in the distal end face of the medicinal liquid container 14. Each of the first fitting claws 21 is composed of a leg section 21 a extending toward the distal end from the distal end face of the medicinal liquid container 14, and a claw section 21 b extending from the tip of the leg section 21 a toward the side of the outer circumferential surface of the medicinal liquid container 14. Thus, the first fitting claws 21 are L-shaped, and, in this embodiment, there are formed integrally with the medicinal liquid container 14.

The first fitting holes 26 are provided in opposed two locations in the base section 22 of the needle support section 8 located at the outer circumference of the fixation section 23. Each of the first fitting holes 26 is composed of an insertion hole 26a which is so formed as to permit the first fitting claw 21 to be inserted thereinto from the proximal end face of the base section 22 and which has a depth for accommodating the first fitting claw 21 therein, and a fitting groove 26b formed over an angular range of 90 degrees clockwise from the insertion hole 26a, with the center of the needle support section 8 as an axis of rotation. The fitting groove 26b is so formed that the claw section 21 b of the first fitting claw 21 can slide therein. When the first fitting claw 21 rotatingly moves while sliding within the fitting groove 26b, an upper portion of the claw section 21 b is blocked by a flange-like part composed of a part of the base section 22.

Thus, when the first fitting claw 21 is rotated along the fitting groove 26b, the claw section 21 b is blocked by the flange-like part composed of a part of the base section 22, so that the first fitting claw 21 is prevented from slipping off to the tip side. Besides, in this case, the medicinal liquid container 14 can be prevented from being disengaged from the needle support section 8.

The second fitting claws 24 are provided in opposed two locations in the outer circumferential side surface of the base section 22, and are formed to project radially outward from the outer circumferential side surface of the base section 22. The second fitting claws 24 are formed integrally with the base section 22. The diameter of the base section 22 at the positions including the second fitting claws 24 (in other words, the maximum diameter of the base section 22) is set to be smaller than the diameter of the inner circumference of the outer tube 2.

The second fitting holes 27 are provided in opposed two locations in a side surface of a recess 18 in a mount section main body 15. Each of the second fitting holes 27 is composed of an insertion hole 27a which is so formed as to permit the second fitting claw 24 to be inserted thereinto from the proximal end face of the mount section main body 15 and which is provided in a shape recessed radially outward from the side surface of the recess 18, and a fitting groove 27b formed over an angular range of 90 degrees counterclockwise from the insertion hole 27a, with the center of the mount section 3 as an axis of rotation. The depth of the insertion holes 27a is the same as the depth of the recess 18. In addition, the fitting groove 27b is so formed that the second fitting claw 24 can slide therein. When the second fitting claw 24 rotatingly moves while sliding within the fitting groove 27b, an upper portion of the second fitting claw 24 is blocked by a flange-like part composed of a part of the mount section 3.

When the second fitting claw 24 is rotated along the fitting groove 27b, the second fitting claw 24 is blocked by the flange-like part composed of the mount section 3, so that the second fitting claw 24 is prevented from slipping off to the tip side. Besides, in this case, the needle support section 8 can be prevented from being disengaged from the mount section 3 to the distal end or being inclined.

In the prefilled syringe 20 configured as above, in the unused state, as shown in FIG. 6A, the second fitting claws 24 formed at the base end 22 have been inserted into the insertion holes 27a in the mount section 3 and, thereafter, the needle support section 8 has been rotated by 90 degrees counterclockwise relative to the mount section main body 15. Thus, in this state, the second fitting claws 24 formed at the base section 22 are in the state of being connectingly fitted in the fitting grooves 27b of the second fitting holes 27 formed in the mount section main body 15, so that the second fitting claws 24 would not slip off to the tip side. Therefore, the needle support section 8 is prevented from being inclined or tilted down. Consequently, the prefilled syringe 20 can be simply handled before use.

Incidentally, in this embodiment, also, the mount section 3 is provided with the first vent hole 3b as shown in the first embodiment; however, the first vent hole 3b is omitted from drawing.

### [2-2 Using Method]

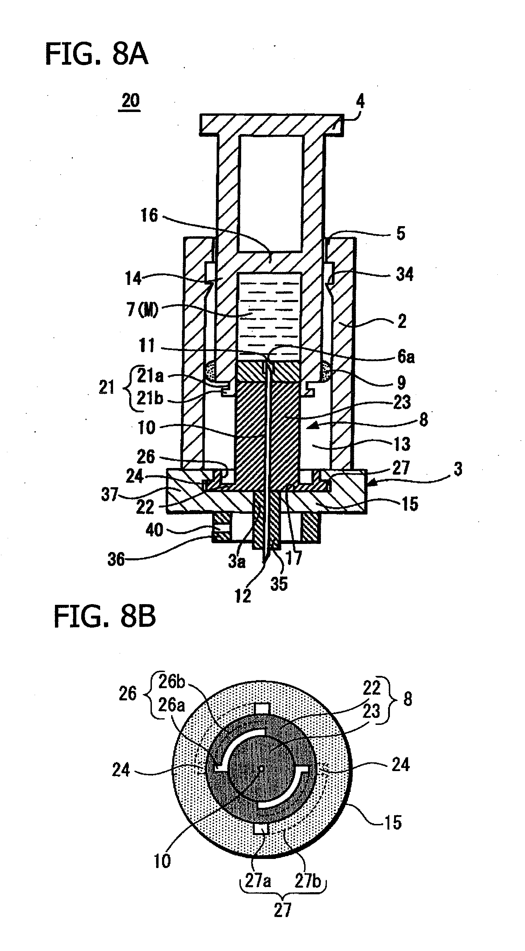

Next, an example of the method of using the prefilled syringe 20 according to this embodiment will be described below. FIGS. 8A, 9A, 10A and 11 are schematic sectional configuration views showing the prefilled syringe 20 in use, whereas FIGS. 8B, 9B and 10B are plan configuration views of a major part in the respective states. In FIGS. 8 to 11, a living body is omitted from drawing, and descriptions of the same steps as those in the first embodiment will be omitted.

At the time of using the prefilled syringe 20, first, the protective member 19 is detached from the distal end of the needle support section 8, and the first needlepoint 12 on the distal end of the double-ended needle 10 is allowed to puncture a part into which a medicinal liquid is to be administered, such as an arm. Then, with a finger put on the pressing section 4, the pressing section 4 is pressed in the distal direction, thereby operating and moving the medicinal liquid container. In this case, the medicinal liquid container 14 is smoothly slid along the inner surface of the outer tube 2, with the seal member 9 therebetween.

As a result, the medicinal liquid container 14 is moved within the outer tube 2 toward the distal end along the center axis, and the second needlepoint 11 on the proximal end of the double-ended needle 10 pierces through the sealing body 6 provided on the distal end in the medicinal liquid container 14, to communicate with the inside of the medicinal liquid container 14. This results in completion of passage of the medicinal liquid into the double-ended needle 10. In this instance, as shown in FIG. 8B, the fitting connection between the needle support section 8 and the mount section 3 is the same as that in the unused state.

After the passage of the medicinal liquid into the double-ended needle 10 is completed, the pressing section 4 is pressed further in the distal direction as shown in FIG. 8A, whereby the sealing body 6 is brought into contact with the fixation section 23 and is slid to move within the medicinal liquid container 14 in the proximal direction. As a result, the medicinal liquid M in the medicinal liquid container 14 is allowed to pass though the double-ended needle 10, and is discharged through the first needlepoint 12 on the distal end, to be administered into a target part.

When the fixation section 23 slides the sealing body 6 toward the side of the proximal end of the medicinal liquid container 14 during the medicinal liquid administration, the medicinal liquid container 14 is moved toward the distal end relative to the fixation section 23 and, therefore, the fixation section 23 is gradually inserted into the inside of the medicinal liquid container 14. Then, the medicinal liquid container 14 is operated and moved so that the first fitting claws 21 provided on the medicinal liquid container 14 are inserted into the insertion holes 26a of the first fitting holes 26 provided in the needle support section 8.

Then, when the sealing body 6 comes into contact with the bottom portion 16 of the medicinal liquid container 14 and the medicinal liquid administration is thereby finished, the fixation section 23 is in the state of being fitted in that part of the medicinal liquid container 14 in which the medicinal liquid M has been stored. Simultaneously with this, the first fitting claws 21 become inserted in the insertion holes 26a of the first fitting holes 26.

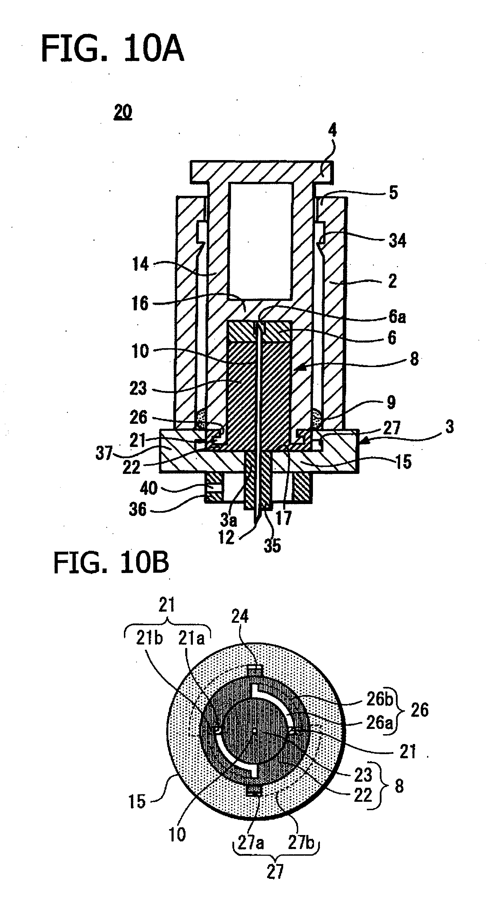

After the medicinal liquid administration is finished, the first needlepoint 12 having punctured the living body is drawn out of the living body. Then, the medicinal liquid container 14 is rotated by 90 degrees clockwise relative to the outer tube 2. As a result, since the fitting grooves 26b of the first fitting holes 26 are formed over the angular range of 90 degrees clockwise from the insertion holes 26a, the rotation is stopped at the point where the claw section 21 b of the first fitting claws 21 have been rotated by 90 degrees within the fitting grooves 26b of the first fitting holes 26. As a result, the medicinal liquid container 14 is connectingly fitted in the needle support section 8, and the medicinal liquid container 14 is locked so as not to be disengaged from the needle support section 8. Next, in the condition where the medicinal liquid container 14 is locked on the needle support section 8, further, the medicinal liquid container 14 is rotated by 90 degrees clockwise. Then, since the medicinal liquid container 14 cannot be rotated further relative to the needle support section 8, this time the needle support section 8 itself is rotated relative to the mount section 3, and is stopped when having been rotated by 90 degrees. When the needle support section 8 is rotated by 90 degrees relative to the mount section main body 15, as shown in FIGS. 10A and 10B, the second fitting claws 24 of the needle support section 8 are located in the insertion holes 27a of the second fitting holes 27 formed in the mount section 3. In this instance, the second fitting claws 24 are exposed at the insertion holes 27a of the second fitting holes 27, whereby the fitting connection between the mount section main body 15 and the needle support section 8 is released, and the needle support section 8 becomes movable at the distal end thereof away from the mount section main body 15.

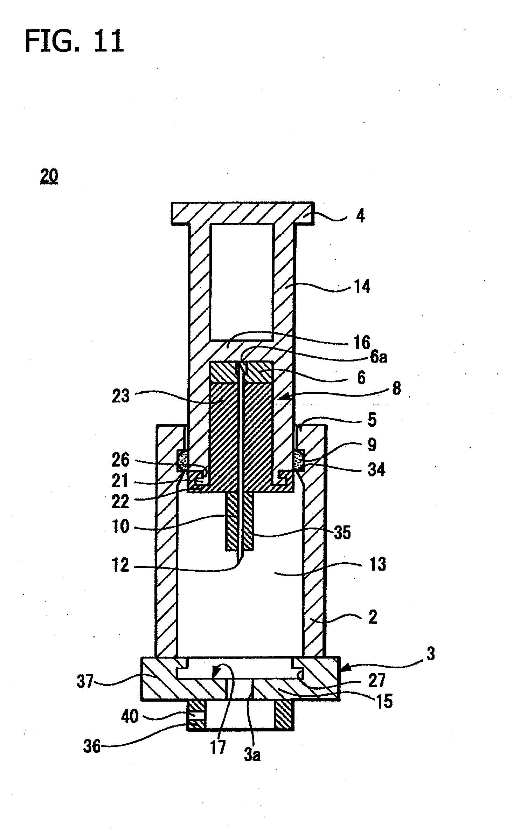

In this condition, the pressing section 4 is pulled toward the proximal end relative to the outer tube 2, as shown in FIG. 11, whereby the medicinal liquid container 14 is operated and moved toward the side of the proximal end of the outer tube 2. This results in that, since the medicinal liquid container 14 and the needle support section 8 are in fitting connection with each other by the first fitting claws 21 and the first fitting holes 26, the medicinal liquid container 14 is moved toward the side of the proximal end of the outer tube 2 and, simultaneously, the needle support section 8 is also moved toward the side of the proximal end of the outer tube 2. As a result, the double-ended needle 10 is drawn out of the mount section main body 15, into the space 13 inside the outer tube. Finally, the first needlepoint 12 of the double-ended needle 10 that had punctured the living body is accommodated into the space 13 inside the outer tube.

Thereafter, in the same manner as in the first embodiment, the pressing section 4 is pulled toward the proximal end until the seal member 9 is disengaged from the locking claw 34 therethrough. The operation of moving the medicinal liquid container 14 is stopped at the time when the seal member 9 has just been disengaged from the locking claw 34.

As has been described above, in the prefilled syringe 20 according to this embodiment, a configuration is adopted wherein in the unused state, the second connection section composed of the second fitting claws 24 and the second fitting holes 27 prevents the needle support section 8 from moving toward the distal end relative to the mount section 3. This prevents the needle support section 8 from being disengaged from the mount section 3, and prevents the double-ended needle 10 supported by the needle support section 8 from being inclined. Consequently, it is possible to secure reliability of the product at the time of use thereof.

In addition, after use, the first connection section composed of the first fitting claws 21 and the first fitting holes 26 keeps the needle support section 8 and the medicinal liquid container 14 in fitting connection with each other. When the medicinal liquid container 14 is operated and moved toward the proximal end, therefore, the needle support section 8 can be assuredly pulled out to the proximal end. In other words, even where the fitting force between the outer circumferential surface of the fixation section 3 and the inner circumferential surface of the medicinal liquid container 14 is weak, the connection between both the members can be achieved. Besides, even after the needle support section 8 is drawn out, the needle support section 8 would not return to the distal end from the inside of the medicinal liquid container 14. As a result, the first needlepoint 12 after use can be securely accommodated into the space 13 inside the outer tube; further, it can be held fixed inside the medicinal liquid container 14. Consequently, enhanced safety is realized.

In addition, the same effects as those of the first embodiment can be obtained.

While an example wherein fitting connection is realized by use of the fitting claws and the fitting holes has been shown as the configuration for the first connection section and the second connection section in this embodiment, a configuration may also be adopted in which two members to be connected together are formed with screw engagement sections (screw sections) and these sections are put into screw engagement.

### <3. Third Embodiment: Prefilled Syringe>

Next, a prefilled syringe according to a third embodiment of the present invention will be described below.

### [3-1 Configuration]

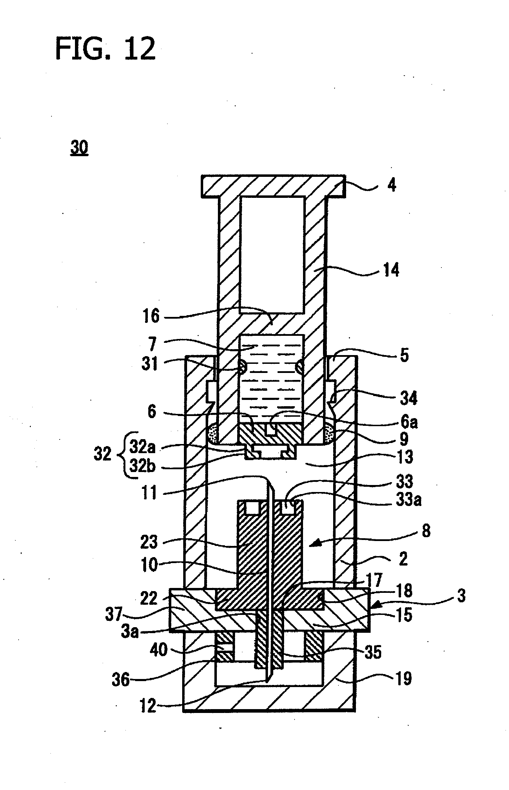

First, the configuration of a prefilled syringe 30 according to this embodiment will be described. FIG. 12 is a schematic sectional configuration view showing the prefilled syringe 30 according to this embodiment in an unused state. In FIG. 12, the parts corresponding to those in FIG. 1 are denoted by the same letters or numerals as used above, and overlapping descriptions of the parts will be omitted.

As shown in FIG. 12, the prefilled syringe 30 in this embodiment includes a connection section by which a sealing body 6 and a needle support section 8 can be connected, and a sealing body locking claw 31 for holding the sealing body 6 on the side of a bottom portion 16 in the inside of a medicinal liquid container 14.

The connection section for connection between the sealing body 6 and the needle support section 8 is composed of fitting claws 32 formed at the distal end face of the sealing body 6, and fitting holes 33 formed in the proximal end face of a fixation section 23 of the needle support section 8.

The fitting claws 32 are provided in opposed two locations in the distal end face of the sealing body 6. Each of the fitting claws 32 is composed of a leg section 32a extending toward the distal end from the distal end face of the sealing body 6, and a claw section 32b extending toward the side of the inner circumferential surface of the sealing body 6 from the tip of the leg section 32a. In other words, the fitting claw 32 is L-shaped. In this embodiment, the fitting claws 32 are formed integrally with the sealing body 6.

The fitting holes 33 are provided in opposed two locations in the proximal end face of the fixation section 23. Each of the fitting holes 33 is composed of an insertion hole 33a which is so formed as to permit the fitting claw 32 to be inserted thereinto from the proximal end face of the fixation section 23 and which has a depth for accommodating the fitting claw 32 therein, and a fitting groove, not shown, which is formed over an angular range of 90 degrees clockwise from the insertion hole 33a, with the center of the fixation section 23 as an axis of rotation. Thus, the configuration of the fitting holes 33 is the same as that of the first fitting holes 26. Therefore, a configuration is realized wherein when the fitting claw 32 is rotated along the fitting groove, the claw section 32b is blocked by the flange-like part composed of a part of the fixation section 23, so that the fitting claw 32 would not slip off to the tip side. As a result, upon fitting connection between the sealing body 6 and the fixation section 23, the sealing body 6 can be prevented from being disengaged from the fixation section 23.

The sealing body locking claw 31 is projectingly provided on the inner circumferential surface of the medicinal liquid container 14, and is configured to be located on the distal end relative to the distal end face of the sealing body 6 when the sealing body 6 is located to make contact with the bottom portion of the medicinal liquid container 14 after medicinal liquid administration.

Incidentally, in this embodiment, also, the mount section 3 is provided with the first vent hole 3b shown in the first embodiment; however, the first vent hole 3b is omitted from drawing.

### [3-2 Using Method]

Next, an example of the method of using the prefilled syringe 30 according to this embodiment will be described below.

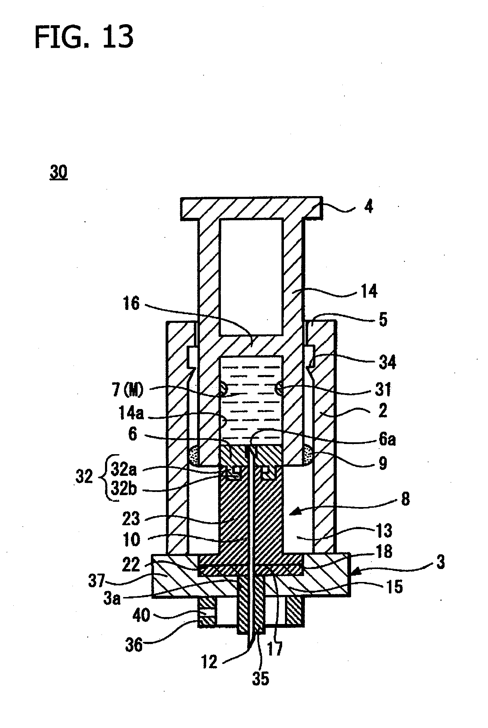

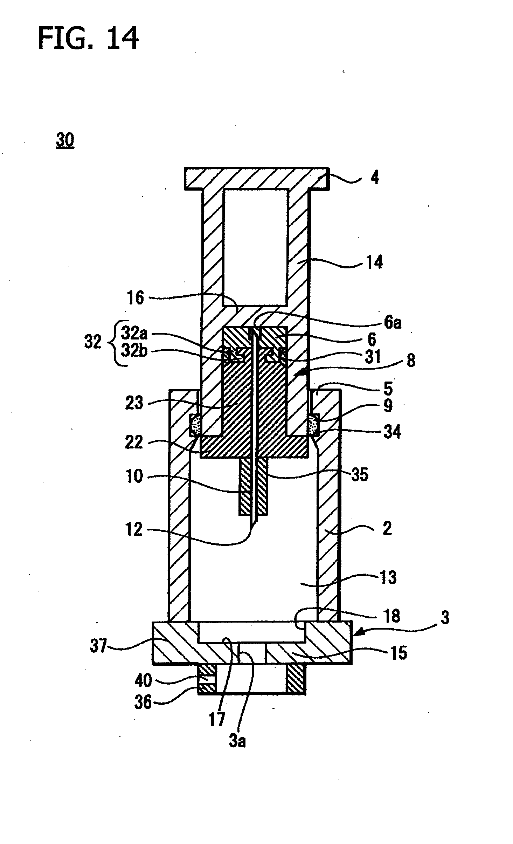

FIGS. 13 and 14 shows schematic sectional configuration views showing the prefilled syringe 30 in use. In FIGS. 13 and 14, a living body is omitted from drawing, and descriptions of the same steps as those in the first embodiment will be omitted.

At the time of using the prefilled syringe 30, first, a protective member 19 is detached from the distal end of the needle support section 8 of the prefilled syringe 30 prepared, and the first needlepoint 12 on the distal end of the double-ended needle 10 is allowed to puncture a part into which a medicinal liquid M is to be administered, such as an arm. Then, with a finger put on the pressing section 4, the pressing section 4 is pressed in the distal direction, thereby operating and moving the medicinal liquid container 14. In this case, the medicinal liquid container 14 is smoothly slid along the inner surface of the outer tube 2, with the seal member 9 therebetween.

As a result, the medicinal liquid container 14 is moved within the outer tube 2 toward the distal end along the center axis. Then, the fitting claws 32 of the sealing body 6 provided on the distal end of the medicinal liquid container 14 are inserted into the insertion holes 33a of the fitting holes 33 in the fixation section 23. In addition, the second needlepoint 11 on the proximal end of the double-ended needle 10 pierces through the sealing body 6, to communicate with the inside of the medicinal liquid container 14. This results in completion of passage of the medicinal liquid into the double-ended needle 10.

After the passage of the medicinal liquid into the double-ended needle 10 is completed, the pressing section 4 is pressed further in the distal direction, whereby the sealing body 6 is brought into contact with the fixation section 23 and is moved within the medicinal liquid container 14 in the proximal direction. As a result, the medicinal liquid M in the medicinal liquid container 14 is allowed to pass through the double-ended needle 10, and is discharged through the first needlepoint 12 on the distal end, to be administered into a target part.

When the fixation section 23 slides the sealing body 6 toward the side of the proximal end of the medicinal liquid container 14 during the medicinal liquid administration, the medicinal liquid container 14 is moved toward the distal end relative to the fixation section 23 and, therefore, the fixation section 23 is gradually inserted into the inside of the medicinal liquid container 14. Then, when the proximal end face of the sealing body 6 comes into contact with the bottom portion 16 and the medicinal liquid administration is thereby finished, the proximal end face of the sealing body 6 is maintained at a fixed position within the medicinal liquid container 14 by the sealing body locking claw 31 of the sealing body 6.

After the medicinal liquid administration is finished, the first needlepoint 12 having punctured the living body is drawn out of the living body. Thereafter, the medicinal liquid container 14 is rotated by 90 degrees clockwise, whereby the fitting claws 32 formed on the sealing body 6 are rotated within the fitting grooves, not shown, of the fitting holes 33, resulting in fitting connection between the sealing body 6 and the fixation section 23. Consequently, the sealing body 6 is locked so as not to be disengaged from the fixation section 23.

Next, the pressing section 4 is pulled toward the proximal end relative to the outer tube 2, whereby the medicinal liquid container 14 is operated and moved toward the side of the proximal end of the outer tube 2. This results in that, since the sealing body 6 and the fixation section 23 are in fitting connection with each other by the connection section composed of the fitting claws 32 and the fitting holes 23, the medicinal liquid container 14 is moved toward the side of the proximal end of the outer tube 2 and, simultaneously, the needle support section 8 is also moved toward the side of the proximal end of the outer tube 2, as shown in FIG. 14. As a result, the double-ended needle 10 is drawn out of the mount section 3, into the space 13 inside the outer tube. Finally, the first needlepoint 12 of the double-ended needle 10 that has punctured the living body is accommodated into the space 13 inside the outer tube.

Thereafter, in the same manner as in the first embodiment, the pressing section 4 is pulled toward the proximal end until the seal member 9 is disengaged from the locking claw 34. The operation of moving the medicinal liquid container 14 is stopped at the time when the seal member 9 has just been disengaged from the locking claw 34.

With the prefilled syringe 30 in this embodiment, also, the same effects as those in the first and second embodiments can be obtained.

Incidentally, the configurations of the first to third embodiments described above can be appropriately combined, and various modifications are possible.

As has been described above, according to the present invention, in a prefilled syringe wherein a double-ended needle and a medicinal liquid container are preliminarily held, a needlepoint after use can be accommodated into a space inside an outer tube safely and simply, and the needlepoint after use can be prevented from being exposed to the exterior. Therefore, erroneous stinging and infection due to erroneous stinging can be prevented from occurring. Consequently, enhanced safety can be realized.

### EXPLANATIONS OF LETTERS OR NUMERALS

1, 20, 30: Prefilled syringe, 2: Outer tube, 3: Mount section, 4: Pressing section, 5: Stopper section, 6: Sealing body, 7: Space, 8: Needle support section, 9: Seal member, 10: Double-ended needle, 11: Second needlepoint, 12: First needlepoint, 13: Space inside outer tube, 14: Medicinal liquid container, 15: Mount section main body, 16: Bottom portion, 17: Mount surface, 18: Recess, 19: Protective member, 22: Base section, 23: Fixation section, 34: Locking claw, 35: Adjustment section, 36: Stabilization section, 37: Guide section

## Claims

1. A prefilled syringe comprising:
an outer tube having a distal end and a proximal end;
a medicinal liquid container which has a distal end and a proximal end, has an outside diameter smaller than the inside diameter of the outer tube, and has a distal opening at the distal end thereof, with the distal opening sealed with a sealing body, whereby a medicinal liquid is sealed in the inside of the container, and with the container movable in an axial direction within the outer tube;
a pressing section by which to operate and move the medicinal liquid container;
a double-ended needle which is provided at one end thereof with a needlepoint capable of puncturing a living body, and is provided at the other end thereof with an end portion capable of puncturing the sealing body;
a needle support section which supports the double-ended needle, is provided in the inside of the outer tube, and can be fitted in the inside of the medicinal liquid container;
a mount section which is provided to cover a proximal end face of the outer tube, and, in an initial state, through which the double-ended needle is passed, and which permits the needle support section to be mounted on the side thereof facing the inside of the outer tube; and
a seal member which seals between the outer tube and the medicinal liquid container,
wherein in the initial state, the medicinal liquid container is held in such a position that the other end of the double-ended needle does not puncture the sealing body,
in a used state, the pressing section is pressed, whereby the medicinal liquid container is moved toward the side of the distal end of the outer tube to cause the other end of the double-ended needle to puncture the sealing body, to slide the sealing body toward the side of the proximal end of the medicinal liquid container, and to fit the needle support section into the inside of the medicinal liquid container, and
in a needle accommodation state, the pressing section is operated, whereby the medicinal liquid container and the needle support section in mutually fitted state are moved together toward the side of the proximal end of the outer tube, and the needlepoint formed at the one end of the double-ended needle is accommodated in the inside of the outer tube.

2. The prefilled syringe according to claim 1,
wherein the seal member is provided fixedly to an outer circumferential surface of the medicinal liquid container, and
the outer tube is provided at an inner circumferential surface with a locking claw which is projectingly provided at a position on the proximal end relative to the position of the seal member in the initial state and which locks the seal member to the proximal end when the medicinal liquid container is moved toward the side of the proximal end of the outer tube.

3. The prefilled syringe according to claim 1,
wherein the medicinal liquid container and the needle support section are provided with connection sections which can be connected to each other.

4. The prefilled syringe according to claim 1,
wherein the needle support section and the mount section are provided with connection sections which can be connected to and disconnected from each other.

5. The prefilled syringe according to claim 1,
wherein the sealing body and the needle support section are provided with connection sections which can be connected to each other.

6. The prefilled syringe according to claim 1,
wherein the outer tube is provided at an inner circumferential surface on the proximal end thereof with a stopper section which is projectingly provided between the outer tube and the medicinal liquid container and which does not permits the seal member to pass therethrough.

7. The prefilled syringe according to claim 4,
wherein the connection sections for connecting the medicinal liquid container and the needle support section are composed of a fitting claw formed on the medicinal liquid container and a fitting hole formed in the needle support section,
the fitting hole is composed of an insertion hole for inserting the fitting claw therein, and a fitting groove by which the fitting claw inserted in the insertion hole is slid in a circumferential direction and which locks the fitting claw, and
connection between the fitting claw and the fitting hole is conducted by locking the fitting claw in the fitting groove through rotating the fitting claw by a predetermined rotational angle within the fitting groove after the fitting claw is inserted into the insertion hole.

8. The prefilled syringe according to claim 5,
wherein the connection sections for connecting the needle support section and the mount section are composed of a fitting claw formed on the needle support section and a fitting hole formed in the mount section,
the fitting hole is composed of an insertion hole for inserting the fitting claw therein, and a fitting groove by which the fitting claw inserted in the insertion hole is slid in a circumferential direction and which locks the fitting claw,
connection between the fitting claw and the fitting hole is conducted by locking the fitting claw in the fitting groove through rotating the fitting claw by a predetermined rotational angle within the fitting groove after the fitting claw is inserted into the insertion hole, and
disconnection of the fitting claw and the fitting hole from each other is conducted by rotating the fitting claw by a predetermined rotational angle within the fitting groove and disengaging the fitting claw from the insertion hole.
